Europäisches Patentamt

European Patent Office

Office européen des brevets

(19)

(11) Veröffentlichungsnummer: **0 268 996**
**B1**

(12) EUROPÄISCHE PATENTSCHRIFT

(45) Veröffentlichungstag der Patentschrift: **06.02.91**

(21) Anmeldenummer: **87117061.9**

(22) Anmeldetag: **19.11.87**

(51) Int. Cl.⁵: **C 07 G 17/00, C 07 D 307/62,**
**C 06 B 31/02, C 06 B 31/28**

(54) **Lacton-Abbauprodukt, Verfahren zu dessen Herstellung und dessen Verwendung.**

(30) Priorität: **27.11.86 CH 4741/86**

(43) Veröffentlichungstag der Anmeldung:
**01.06.88 Patentblatt 88/22**

(45) Bekanntmachung des Hinweises auf die
Patenterteilung:
**06.02.91 Patentblatt 91/06**

(84) Benannte Vertragsstaaten:
**AT BE CH DE FR GB IT LI NL SE**

(56) Entgegenhaltungen:
**WO-A-86/02347**
**US-A-4 497 676**

**JOURNAL OF AGRICULTURAL AND FOOD
CHEMISTRY, Band 17, Nr. 1, Januar-Februar
1969, Seiten 38-40, Washington, DC, US; J.H.
TATUM et al.: "Degradation products from
ascorbic acid"**

(73) Patentinhaber: **F. HOFFMANN-LA ROCHE AG**
**Postfach 3255**
**CH-4002 Basel (CH)**

(72) Erfinder: **Beyeler, Paul, Dr.**
**Düchelweiher 4**
**CH-4144 Arlesheim (CH)**
Erfinder: **Fürbringer, Claude**
**Unterm Schellenberg 80**
**CH-4125 Riehen (CH)**

(74) Vertreter: **Aschert, Hubert et al**
**Grenzacherstrasse 124 Postfach 3255**
**CH-4002 Basel (CH)**

## EP 0 268 996 B1

**Beschreibung**

Die vorliegende Erfindung betrifft ein Lacton-Abbauprodukt, und zwar ein Abbauprodukt von Ascorbinsäure bzw, Isoascorbinsäure, sowie ein Verfahren zur Herstellung eines derartigen Abbauprodukts. Die vorliegende Erfindung betrifft ferner die Verwendung eines solchen Abbauproduktes zur Herstellung von Explosivstoffen (Sprengstoffen, Schiess- oder Treibstoffen und pyrotechnischen Stoffen), sowie ein Verfahren zur Herstellung solcher Explosivstoffe unter Verwendung eines solchen Abbauprodukts und schliesslich so erhältliche Explosivstoffe.

Aus US—A—4 497 676 und WO—A—86 02347 ist es bekannt, Explosivstoffe dadurch herzustellen, dass man Ascorbinsäure oder Isoascorbinsäure zusammen mit einem Nitrat-haltigen Oxidationsmittel auf eine etwa 75°C nicht übersteigende Temperatur erhitzt.

Es wurde nun gefunden, dass man Explosivstoffe ähnlicher Art in sicherer Weise dadurch herstellen kann, dass man ein durch Erhitzen von Ascorbinsäure oder Isoascorbinsäure primär erhaltenes Abbauprodukt in Abwesenheit eines Nitrat-haltigen Oxidationsmittels auf bestimmte Temperaturen erhitzt und dieses Abbauprodukt dann mit dem Nitrat-haltigen Oxidationsmittel vermischt.

Das genannte Abbauprodukt wird erfindungsgemäss dadurch hergestellt, dass man Ascorbinsäure oder Isoascorbinsäure in Abwesenheit eines Nitrat-haltigen Oxidationsmittels auf eine Temperatur zwischen etwa 70°C und etwa 210°C erhitzt bis etwa 10—100% der eingesetzten Säure abgebaut werden, wobei im Falle der Verwendung der Säure in Form einer Lösung, in Form einer Suspension oder in feuchtem Zustand die Lösung bzw. Suspension bzw. das feuchte Material zur Trockene eingedampft wird.

Gemäss einer zweckmässigen Ausführungsform des erfindungsgemässen Verfahrens erfolgt die Erhitzung auf eine Temperatur zwischen etwa 80°C und 190°C, vorzugsweise 80°C bis 150°C.

Die Zeitdauer der Erhitzung richtet sich nach der angewandten Erhitzungstemperatur, sowie nach der zur Erhitzung verwendeten Einrichtung. Im allgemeinen wird bei einer hohen Erhitzungstemperatur die Zeitdauer der Erhitzung verhältnismässig kurz und bei niedrigerer Erhitzungstemperatur verhältnismässig länger sein. Die Erhitzungszeit kann im allgemeinen zwischen etwa einer Minute und mehreren Stunden, vorzugsweise zwischen etwa einer Minute und 3 Stunden liegen. Verhältnismässig kurze Erhitzungszeiten, beispielsweise 1 Minute bis etwa 60 Minuten, sind bei Verwendung von Mikrowellenöfen oder Extrudern möglich.

Die Erhitzung kann gegebenenfalls unter Luftausschluss erfolgen.

Die Erhitzung der Säure kann in festem Zustand oder in flüssigem Zustand erfolgen, vorzugsweise wird die Erhitzung jedoch in Lösung, zweckmässigerweise in wässriger Lösung, bzw. in Suspension, zweckmässig in wässriger Suspension durchgeführt, um eine möglichst gleichmässige Erhitzung des gesamten zu erhitzenden Materials zu erzielen. Ferner ist es möglich, die Erhitzung der Säure in feuchtem Zustand, vorzugsweise in wasserfeuchtem Zustand durchzuführen. Sowohl bei der Erhitzung in Lösung, als auch bei der Erhitzung in Suspension und bei der Erhitzung in feuchtem Zustand wird das Lösungs- bzw. Suspensionsmittel während der Erhitzung abgedampft. Hierbei entweicht auch Kohlendioxyd.

Nach einer bevorzugten Ausführungsform des erfindungsgemässen Verfahrens wird die Erhitzung in Gegenwart einer den Abbau der Säure begünstigenden Substanz, nämlich eines Alkali- oder Erdalkalisalzes, insbesondere in Gegenwart des Natrium- oder Kaliumsalzes der Ascorbinsäure oder Isoascorbinsäure durchgeführt. Diese Salze können auch in situ, durch Zugabe des entsprechenden Hydroxyds, Carbonats oder Bicarbonats zur Säure hergestellt werden.

Das Salz kommt zweckmässig in einer Menge von etwa 0,1 bis 100 Gew.%, vorzugsweise in einer Menge von 0,5 bis 50 Gew.%, insbesondere in einer Menge von etwa 1 bis 10 Gew.% zur Anwendung. Die Abbaugeschwindigkeit wird durch die Menge an verwendetem Ascorbat beeinflusst, in dem Sinne, dass höhere Mengen Ascorbat zu einem rascheren Abbau führen.

Das einen weiteren Gegenstand der vorliegenden Erfindung bildende Abbauprodukt von Ascorbinsäure bzw. Isoascorbinsäure ist ein Produkt, welches gemäss dem obigen Verfahren erhältlich ist, d.h. ein Produkt, welches dadurch erhältlich ist, dass man die Ascorbinsäure bzw. Isoascorbinsäure, in Abwesenheit eines Nitrat-haltigen Oxidationsmittels auf eine Temperatur zwischen etwa 70°C und etwa 200°C erhitzt, wobei etwa 10 bis 100% der eingesetzten Säure abgebaut werden.

Ein derartiges Produkt ist, in Abhängigkeit von der angewandten Erhitzungstemperatur, der angewandten Erhitzungszeit, der Art und Menge der verwendeten den Abbau der Säure begünstigenden Substanz im wesentlichen durch die folgenden Eigenschaften bzw. Parameter gekennzeichnet:

Braunes, zumindest teilweise wasserlösliches Pulver, enthält neben nicht abgebauter Säure polymere Substanzen, Furanone und Furanole, sowie andere Abbau- bzw. Umwandlungsprodukte der Ausgangsverbindung; Abbaugrad 10—100%, beispielsweise 40—90% und 60—90%, vorzugsweise 65—85%; Gewichtsverlust gegenüber Ausgangsmaterial 5—40%, insbesondere 10—30%; Verbrennungswärme: 3315 cal/g bei einem Abbaugrad von 50%, 3394 cal/g bei einem Abbaugrad von 65% und 4200 cal/g und mehr bei noch höheren Abbaugraden.

Wie eingangs ausgeführt wurde, eignet sich das nach dem erfindungsgemässen Verfahren erhältliche Abbauprodukt zur Herstellung von Explosivstoffen, wobei dieses Abbauprodukt mit einem Nitrat-haltigen Oxidationsmittel vermischt wird, wobei in die Mischung noch weitere Zusatzstoffe, beispielsweise Gelatinierungsmittel oder Stabilisatoren, wie substituierte Harnstoffe, z.B. Akardit® oder Centralit®; substituierte Urethane, Phthalate, Polymere, Zusätze zu Leuchtsätzen, wie Natrium-, Barium-, Strontium-oder Kupfersalze, sowie andere Additive, z.B. zur Erhöhung der Explosionsenergie oder zur Verbesserung anderer wünschenswerter Eigenschaften, wie beispielsweise Bor oder Nitroguanidin, eingearbeitet werden können.

Als Nitrat-haltiges Oxidationsmittel wird vorzugsweise ein Alkali- oder Erdalkalinitrat oder Ammoniumnitrat verwendet, wobei auch Gemische solcher Nitrate angewandt werden können.

Weitere Nitrat-haltige Oxidationsmittel sind organische Nitrate. Unter einem organischen Nitrat ist hierbei jedes übliche, kohlenstoffhaltige Nitrat mit einem stoechiometrischen Sauerstoffüberschuss zu verstehen, welches üblicherweise in der pyrotechnischen Industrie oder in der Triebstoffindustrie verwendet wird. Beispiele geeigneter organischer Nitrate sind Nitrocellulose, Nitroglycerin und Pentaerythrit-tetranitrat.

Das Gewichtsverhältnis Nitrat-haltiges Oxidationsmittel:Abbauprodukt kann zwischen 90:10 und 50:50, vorzugsweise zwischen 80:20 und 60:40 schwanken, und zwar je nach Anwendungszweck des Endproduktes. Diese Verhältniszahlen beziehen sich auf Kaliumnitrat als Nitrat-haltiges Oxidationsmittel.

Zur Herstellung eines Explosivstoffes unter Verwendung des erfindungsgemässen Abbauproduktes und eines Nitrathaltigen Oxidationsmittels werden die beiden Substanzen, gegebenenfalls unter Zusatz anderer Zusatzstoffe miteinander vermischt. Hierbei ist sowohl die trockene Vermischung möglich als auch die Herstellung einer Mischung in einer Lösung, beispielsweise einer wässrigen Lösung mit anschliessendem Eindampfen dieser Lösung. Als besonders vorteilhaft hat sich die Herstellung eines trockenen Endproduktes (Herstellung des Explosivstoffes) mittels Sprühtrocknung aus einer Lösung oder Suspension erwiesen. Hierbei können mittels an sich bekannten Methoden und Vorrichtungen wie beispielsweise Ultraschallvibrationsdüse oder durch geeignete Temperaturführung, unterschiedliche Tropfengrössen und damit verschiedenartige Granulate erhalten werden, wie sie für verschieden Anwendungen geeignet sind. Neben der Sprühtrocknung bietet sich vor allem auch die Trocknung mittels Dünnschichtverdampfer oder Bandtrockner an. Es ist auch die Herstellung des Endproduktes in Form von Granulaten über die Schmelze möglich. Es besteht auch die Möglichkeit, die Erhitzung der Ascorbinsäure bzw. der Isoascorbinsäure in einer Knetvorrichtung durchzuführen und das Oxidationsmittel nach Erreichung des gewünschten Abbaugrades direkt zuzumischen.

Falls erwünscht kann man auch während der Endproduktherstellung (Herstellung des Explosivstoffes) noch einen weiteren Abbau von Ascorbinsäure durchführen, indem man ein Abbauprodukt mit höherem Ascorbinsäuregehalt einsetzt und den Abbau durch thermische Behandlung beim Mischvorgang oder bei der Formulierung des Endproduktes weiterführt.

Ein auf diese Weise erhältliches Endprodukt ist ein Explosivstoff in Form eines Sprengstoffs, z.B. für Verwendung im Bergbau, eines Schiess- oder Treibstoffes oder eines pyrotechnischen Stoffes bzw. eines energiereichen Gemisches, welches für verschiedene Antriebszwecke verwendet werden kann.

Das so erhältliche Endprodukt kann beispielsweise für die Herstellung von Patronen oder Kartuschen. für Leucht- oder Signalmunition, für Raketen, für Schussapparate zu technischen Zwecken, sowie für Feuerwerkskörper oder dergleichen Verwendung finden.

Der so erhältliche Explosivstoff zeichnet sich durch hohe Sicherheit, geringe Korrosivität, hohe Treibkraft und geringe Rauchentwicklung aus.

## Beispiel 1

8,95 g Ascorbinsäure und 1,05 g Kaliumascorbat werden in 50 ml $H_2O$ gelöst und danach am Rollverdampfer bei 45°C/15 mbar bis zur Trockene eingedampft. Anschliessend lässt man das trockene Gemisch in einer Petrischale während 3 Stunden bei 130°C im Heizschrank stehen. Auf diese Weise werden 8,5 g eines Gemisches erhalten, das noch ca. 42% Ascorbinsäure enthält.

## Beispiel 2

In einem analogen Versuch wie in Beispiel 1 wird während 75 Minuten bei 150°C stehen gelassen. Unter diesen Bedingungen erhält man 7 g eines Gemisches mit einem Ascorbinsäuregehalt von ca. 7%.

## Beispiel 3

895 mg Ascorbinsäure und 105 mg Kaliumascorbat werden in einem Glasgefäss mit 0,4 ml Wasser angerührt und dann in einem Mikrowellenofen bei 85°C und 2450 MHz während 5 Minuten stehen gelassen. Auf diese Weise erhält man 990 mg eines Gemisches, das noch etwa 85% Ascorbinsäure enthält.

## Beispiel 4

In einem analogen Versuch wie in Beispiel 3 wird das Gemisch während 10 Minuten im Mikrowellenofen erwärmt. Dies ergibt ca. 700 mg eines Gemisches mit einem Restascorbinsäure-Gehalt von etwa 1%.

In der folgenden Tabelle sind verschiedene Eigenschaften des nach den Beispielen 1 und 2 erhaltenen Abbauproduktes zusammengestellt:

## Tabelle

| | Beispiel 1 Ascorbinsäure auf 42% abgebaut | Beispiel 2 Ascorbinsäure auf 7% abgebaut |
|---|---|---|
| Elementar-analyse | C 43,43 H 5,34 | C 45,82 H 4,91 |
| Fp. | nicht bestimmbar, ab 140°C schäumt die Substanz | gleich wie Beispiel 1 |
| IR KBr | 1117 cm$^{-1}$ Alkohol-Bande<br>1275 Ester, COOH<br>1675 COOH, -C=O<br>1755 5-Ring Lacton | gleich wie Beispiel 1 |
| NMR DMSO | Gemisch, z.T. noch C-Skelett wie in Ascorbin-säure | gleich wie Beispiel 1 |
| $H_2O$ löslich | ca. 25 g/100 ml (25°C) | ca. 25 g/100 ml (25°C) |

### Beispiel 5

700 mg Kaliumnitrat und 300 mg des Abbauproduktes nach Beispiel 1 werden in 10 ml Wasser aufgelöst und unter vermindertem Druck bei 40°C zur Trockene eingedampft. Man erhält 1 g eines Explosivstoffes mit einer Explosionswärme (bei Zündung unter Luftabschluss) von 669 cal/g.

### Beispiel 6

Wenn man gemäss Beispiel 5 verfährt, als Abbauprodukt jedoch das Produkt gemäss Beispiel 2 verwendet, erhält man einen Explosivstoff mit einer Explosionswärme von 700 cal/g.

## Patentansprüche

1. Verfahren zur Herstellung eines Abbauproduktes von Ascorbinsäure bzw. Isoascorbinsäure, dadurch gekennzeichnet, dass man diese Säure in Abwesenheit eines Nitrat-haltigen Oxidationsmittels auf eine Temperatur zwischen etwa 70°C und etwa 210°C erhitzt bis etwa 10—100% der eingesetzten Säure abgebaut werden, wobei im Falle der Verwendung der Säure in Form einer Lösung, in Form einer Suspension oder in feuchtem Zustand die Lösung bzw. Suspension bzw. das feuchte Material zur Trockene eingedampft wird.

2. Verfahren nach Anspruch 1, dadurch gekennzeichnet, dass man, in Abhängigkeit von der Erhitzungstemperatur, während eines Zeitraums von einer Minute bis mehreren Stunden, vorzugsweise von einer Minute bis etwa 3 Stunden erhitzt.

3. Verfahren nach Anspruch 1 oder 2, dadurch gekennzeichnet, dass man die Erhitzung der Säure in Lösung, vorzugsweise in wässeriger Lösung, bzw. in Suspension, bzw. in feuchtem Zustand, vorzugsweise wasserfeuchtem Zustand durchführt, wobei das Lösungs- bzw. Suspensionsmittel während der Erhitzung abgedampft wird.

4. Verfahren nach Anspruch 1, 2 oder 3, dadurch gekennzeichnet, dass man die Erhitzung in Gegenwart einer den Abbau der Säure begünstigenden Substanz, nämlich eines Alkali- oder Erdalkalisalzes durchführt.

5. Verfahren nach Anspruch 4, dadurch gekennzeichnet, dass man die Erhitzung in Gegenwart eines Alkali- oder Erdalkalisalzes der Ascorbinsäure bzw. Isoascorbinsäure, insbesondere des Natrium- oder Kaliumsalzes durchführt.

6. Verfahren nach Anspruch 5, dadurch gekennzeichnet, dass man das Salz in einer Menge von etwa 0,1 bis 100 Gew.%, vorzugsweise in einer Menge von 0,5 bis 50 Gew.%, insbesondere in einer Menge von etwa 1 bis 10 Gew.% verwendet.

7. Abbauprodukt erhältlich nach dem Verfahren nach einem der Ansprüche 1 bis 6.

8. Abbauprodukt nach Anspruch 7, gekennzeichnet durch die folgenden Eigenschaften bzw. Parameter:

Braunes, zumindest teilweise wasserlösliches Pulver, enhält neben nicht abgebauter Säure polymere Substanzen, Furanone und Furanole; Abbaugrad 10—100%, beispielsweise 40—90% und 60—90%, vorzugsweise 65—85%; Gewichtsverlust gegenüber Ausgangsmaterial 5—40%, insbesondere 10—30%; Verbrennungswärme: 3315 cal/g bei einem Abbaugrad von 50% und 3394 cal/g bei einem Abbaugrad von 65%.

9. Verwendung des nach einem der Ansprüche 1 bis 8 erhältlichen Abbauproduktes zur Herstellung von Explosivstoffen durch Vermischen mit einem Nitrat-haltigen Oxidationsmittel.

10. Verwendung nach Anspruch 9, dadurch gekennzeichnet, dass als Nitrat-haltiges Oxidationsmittel ein Alkali- oder Erdalkalimetallnitrat oder Ammoniumnitrat oder Mischungen dieser Nitrate verwendet werden.

11. Verfahren zur Herstellung von Explosivstoffen, dadurch gekennzeichnet, dass man ein nach dem Verfahren gemäss einem der Ansprüche 1 bis 6 erhältliches Abbauprodukt mit einem Nitrat-haltigen Oxidationsmittel und gegebenenfalls mit weiteren Zusätzen vermischt.

12. Verfahren nach Anspruch 11, dadurch gekennzeichnet, dass man als Nitrat-haltiges Oxidationsmittel ein Alkali- oder Erdalkalinitrat oder Ammoniumnitrat oder Gemische dieser Nitrate verwendet.

13. Verfahren nach Anspruch 12, dadurch gekennzeichnet, dass man das Nitrat-haltige Oxidationsmittel in einem Gewichtsverhältnis zum Gewicht des Abbauproduktes von zwischen 90:10 und 50:50, vorzugsweise 80:20 und 60:40, bezogen auf Kaliumnitrat, verwendet.

14. Explosivstoff, dadurch gekennzeichnet, dass er ein nach dem Verfahren gemäss einem der Ansprüche 1—6 erhältliches Abbauprodukt und ein Nitrat-haltiges Oxidationsmittel enthält.

15. Explosivstoff nach Anspruch 14, dadurch gekennzeichnet, dass das Gewichtsverhaltnis von Abbauprodukt und Nitrat-haltigem Oxidationsmittel zwischen 90:10 und 50:50, vorzugsweise zwischen 80:20 und 60:40, bezogen auf Kaliumnitrat, beträgt.

**Revendications**

1. Procédé pour la préparation d'un produit de dégradation de l'acide ascorbique ou de l'acide isoascorbique, caractérisé en ce que l'on chauffe ces acides, en l'absence d'un oxydant nitré, à une température comprise entre environ 70°C et environ 210°C jusqu'à ce que soient dégradés environ 10% à environ 100% de l'acide utilisé, la solution ou la suspension ou le matériau humide, suivant que l'on utilise l'acide sous forme de solution ou de suspension ou à l'état humide, étant évaporé à sec.

2. Procédé selon la revendication 1, caractérisé en ce que l'on chauffe, suivant la température de chauffage, pendant une durée allant d'une minute à plusieurs heures, de préférence allant d'une minute à environ 3 heures.

3. Procédé selon la revendication 1 ou 2, caractérisé en ce que l'on effectue le chauffage de l'acide en solution, de préférence en solution aqueuse ou en suspension ou à l'état humide, de préférence à l'état humide d'eau, le solvant ou l'agent de suspension étant évaporé pendant le chauffage.

4. Procédé selon l'une des revendications 1, 2 ou 3, caractérisé en ce que l'on effectue le chauffage en présence d'une substance favorisant la dégradation de l'acide, à savoir un sel alcalin ou alcalino-terreux.

5. Procédé selon la revendication 4, caractérisé en ce que l'on effectue le chauffage en présence d'un sel alcalin ou alcalino-terreux de l'acide ascorbique ou de l'acide isoascorbique, en particulier du sel de sodium ou de potassium.

6. Procédé selon la revendication 5, caractérisé en ce que l'on utilise le sel en une quantité d'environ 0,1 à environ 100% en poids, de préférence en une quantité de 0,5 à 50% en poids, en particulier en une quantité d'environ 1 à environ 10% en poids.

7. Produit de dégradation obtenu par le procédé selon l'une des revendications 1 à 6.

8. Produit de dégradation selon la revendication 7, caractérisé en ce qu'il présente les propriétés ou paramètres suivants:

Poudre brune, au moins en partie soluble dans l'eau, contenant à côté de l'acide non dégradé des substances polymères furanones et furanols; taux de dégradation 10—100%, par exemple 40—90% et 60—90%, de préférence 65—85%; perte de poids par rapport au matériau de départ 5—40%, en particulier 10—30%; chaleur de combustion: 3 315 cal/g pour un taux de dégradation de 65%.

9. Utilisation du produit de dégradation obtenu selon l'une des revendications 1 à 8 pour la préparation d'explosifs par mélange avec un oxydant nitré.

10. Utilisation selon la revendication 9, caractérisée en ce que l'on utilise, comme oxydant nitré, un nitrate de métal alcalin ou de métal alcalino-terreux ou du nitrate d'ammonium ou des mélanges de ces nitrates.

11. Procédé pour la préparation d'explosifs, caractérisé en ce que l'on mélange un produit de dégradation obtenu par le procédé selon l'une des revendications 1 à 6 avec un oxydant nitré et éventuellement avec d'autres additifs.

12. Procédé selon la revendication 11, caractérisé en ce que l'on utilise, comme oxydant nitré, un nitrate alcalin ou alcalino-terreux ou un nitrate d'ammonium ou des mélanges de ces nitrates.

13. Procédé selon la revendication 12, caractérisé en ce que l'on utilise l'oxydant nitré dans une proportion en poids par rapport au poids du produit de dégradation comprise entre 90:10 et 50:50, de préférence entre 80:20 et 60:40, rapportée au nitrate de potassium.

14. Explosif caractérisé en ce qu'il contient un produit de dégradation obtenu par le procédé selon, l'une des revendications 1 à 6.

15. Explosif selon la revendication 14, caractérisé en ce que la proportion en poids du produit de dégradation et de l'oxydant nitré est située entre 90:10 et 50:50, de préférence entre 80:20 et 60:40, rapportée au nitrate de potassium.

## Claims

1. A process for the manufacture of a degradation product of ascorbic acid or isoascorbic acid, characterized by heating these acidsin the presence of a nitrate-containing oxidation agent to a temperature between about 70°C and about 210°C until about 10—100% of the acid which is used are degraded, whereby when the acid is used in the form of a solution, in the form of a suspension or in moist condition the solution or suspension or the moist material is evaporated to dryness.

2. A process according to claim 1, characterized in that, depending on the heating temperature, the heating is carried out for a period of one minute to several hours, preferably of one minute to about 3 hours.

3. A process according to claim 1 or 2, characterized in that the heating of the acid is carried out in solution, preferably in aqueous solution, or in suspension or in moist condition, preferably water-moist condition, whereby the solution or suspension agent is evaporated during the heating.

4. A process according to claim 1, 2 or 3, characterized in that the heating is carried out in the presence of a substance which promotes the degradation of the acid, namely an alkali or alkaline earth salt.

5. A process according to claim 4, characterized in that the heating is carried out in the presence of an alkali or alkaline earth salt of ascorbic acid or isoascorbic acid, especially the sodium or potassium salt.

6. A process according to claim 5, characterized in that the salt is used in an amount of about 0.1 to 100 wt.%, preferably in an amount of 0.5 to 50 wt.%, especially in an amount of about 1 to 10 wt.%.

7. A degradation product obtainable according to the process according to any one of claims 1 to 6.

8. A degradation product according to claim 7, characterized by the following properties or parameters:

Brown, at least partially water-soluble powder, containing polymeric substances, furanones and furanols in addition to non-degraded acid; degree of degradation 10—100%, for example 40—90% and 60—90%, preferably 65—85%; weight loss compared with starting material 5—40%, especially 10—30%; heat of combustion: 3315 cal/g with a degree of degradation of 50% and 3394 cal/g with a degree of degradation of 65%.

9. The use of a degradation product obtainable according to any one of claims 1 to 8 for the manufacture of explosive materials by admixture with a nitrate-containing oxidation agent.

10. The use according to claim 9, characterized in that an alkali or alkaline earth metal nitrate or ammonium nitrate or mixtures of these nitrates is/are used as the nitrate-containing oxidation agent.

11. A process for the manufacture of explosive materials, characterized by mixing a degradation product obtainable according to the process in accordance with any one of claims 1 to 6 with a nitrate-containing oxidation agent and, if desired, with further additives.

12. A process according to claim 11, characterized in that an alkali or alkaline earth nitrate or ammonium nitrate or a mixture of these nitrates is used as the nitrate-containing oxidation agent.

13. A process according to claim 12, characterized in that the nitrate-containing oxidation agent is used in a weight ratio to the weight of the degradation product of between 90:10 and 50:50, preferably 80:20 and 60:40, based on potassium nitrate.

14. A explosive material, characterized in that it contains a degradation product obtainable according to the process in accordance with any one of claims 1—6 and a nitrate-containing oxidation agent.

15. An explosive material, according to claim 14, characterized in that the weight ratio of degradation product and nitrate-containing oxidation agent is between 90:10 and 50:50, preferably between 80:20 and 60:40, based on potassium nitrate.